# EUROPEAN PATENT APPLICATION

(11) **EP 3 480 300 A1**
(43) Date of publication of application: **08.05.2019**
(21) Application number: 18202295.4
(22) Date of filing: 24.10.2018
(51) Int. Cl.: C12N 5/071

(54) **METHOD FOR PREPARING SAMPLE FOR MICROSCOPE EXAMINATION AND SAMPLE PREPARATION KIT**

(30) Priority: 07.11.2017 JP 2017214301
(71) Applicant: OLYMPUS CORPORATION, Hachioji-shi Tokyo 192-8507 (JP)
(72) Inventor: OGO, Katsunori, Tokyo, 192-8507 (JP)
(74) Representative: Gunzelmann, Rainer

(57) **Abstract**

Provided is a method for preparing a sample for microscope examination, the method including bringing a liquid (B) for gelling or solidifying a medium solution (A) into contact with a surface of the medium solution (A), which contains at least one spheroid (S) and which is substantially transparent when being gelled or solidified, in a state where the liquid (B) is in the form of mist, bubbles, or liquid droplets with a smaller volume than the volume of the medium solution (A), thereby gelling or solidifying the medium solution (A).

## Description

### {Technical Field}

The present invention relates to a method for preparing a sample for microscope examination and a sample preparation kit.

### {Background Art}

Nowadays, there is a growing interest in microscope examination of three-dimensionally cultured cell aggregates such as spheroids and organoids. There are known cell aggregate preparation methods (refer to, for example, Patent Literature 1). In the method described in Patent Literature 1, droplet-shaped culture solutions containing cells therein are dispensed onto the inner surface of the lid of a petri dish, and then these droplets are inverted to form hanging drops. Thereafter, with the help of a component force of gravity in a direction along the curved surfaces of the hanging drops, cell aggregates are developed in the hanging drops. With the method described in Patent Literature 1, however, the hanging drops are not arrayed correctly, and hence it is obvious that this method is not appropriate for automated preparation of cell aggregates.

There is a known multi-well plate structure that is made appropriate for automated formation of hanging drops by improving the technique in Patent Literature 1 (refer to, for example, Patent Literature 2). The multi-well plate described in Patent Literature 2 includes: a hollow section for receiving a liquid discharged from a dispensing burette; a hanging drop formation compartment for forming a hanging drop and holding the formed hanging drop; and a conduit continuing to the hollow section and the hanging drop formation compartment, wherein sets of the hollow section, hanging drop formation compartment, and conduit are arranged in an array. Unlike the method described in Patent Literature 1, the multi-well plate described in Patent Literature 2 does not require the droplets to be inverted, and hanging drops can be formed merely by dispensing cells, culture solutions, and so forth from above the multi-well plate according to the array format, thus facilitating automated preparation of cell aggregates using hanging drops. However, Patent Literature 2, like Patent Literature 1, does not make any mention about a method for highly precise examination with a microscope.

There is a known technique for precise examination and imaging with a microscope that has been achieved by further developing the technique in Patent Literature 2 (refer to, for example, Patent Literature 3). In the technique described in Patent Literature 3, the prepared cell aggregate in a hanging drop is dropped, together with the hanging drop, into a well of a multi-well plate having a flat and transparent bottom surface, and then the light emitted from the cell aggregate is collected by the objective lens of an inverted microscope via the bottom surface of the well, thus performing examination and imaging of the cell aggregate.

### {Citation List}

### {Patent Literature}

{PTL 1}
   German Patent Specification No. 10362002
{PTL 2}
   Publication of Japanese Patent No. 5490803
{PTL 3}
   PCT International Publication No. WO 2017/001680 {Summary of Invention}

### {Technical Problem}

In the technique described in Patent Literature 3, however, a ridge portion, which is the boundary between the bottom surface and a lateral surface of the well, interferes with a light beam that is to be received by the objective lens if the cell aggregate is disposed in contact with the lateral surface of the well, and this hampers the optical performance intrinsic to the microscope. In particular, in the case of examination with a light-sheet microscope, a cell aggregate is irradiated with excitation light from a lateral surface, and therefore, it is very difficult to examine the portion of the cell aggregate in contact with the lateral surface of the well. For this reason, the cell aggregate needs to be fixed to a position where the cell aggregate does not come into contact with the bottom surface of the well.

For more highly precise examination, there is also another demand for gelling a hanging drop containing a cell aggregate and examining the cell aggregate in a state where the hanging drop is placed in a liquid having a refractive index equivalent to that of the hanging drop. However, with a method in which a hanging drop is gelled simply by mixing two or more solutions, the whole solution of the hanging drop is gelled, which destroys the fluidity of the hanging drop. In addition, with a method in which a gelling liquid, such as a solution containing calcium ions, is poured, using a pipette, in a hanging drop containing sodium alginate in the form of a solution has a problem in that the solution is immediately gelled at the tip of the pipette, and hence it is difficult to stir the gelling liquid in the whole hanging drop. Furthermore, with the method in which the hanging drop is gelled by mixing these two or more solutions and the method in which the gelling liquid is poured, using a pipette, in the hanging drop have a problem in that automation using a dispensing burette or a robot is very difficult.

The present invention has been conceived in light of the above-described circumstances, and an object thereof is to provide a method for preparing a sample for microscope examination and a sample preparation kit, the method and the kit allowing not only easy preparation of a sample that enables highly precise examination and imaging of an examination object with a microscope but also easy automation of the preparation of such a sample.

### {Solution to Problem}

In order to achieve the above-described object, the present invention provides the following solutions.

A first aspect of the present invention is a method for preparing a sample for microscope examination, the method including: bringing a liquid for gelling or solidifying a medium solution into contact with a surface of the medium solution, which contains at least one examination object and which is substantially transparent when being gelled or solidified, in a state where the liquid is in the form of mist, bubbles, or liquid droplets with a smaller volume than the volume of the medium solution, thereby gelling or solidifying the medium solution.

According to this aspect, the liquid in the form of mist, bubbles, or liquid droplets with a volume smaller than that of the medium solution comes into contact with the surface of the medium solution containing the examination object, and as a result of the medium solution being gelled or solidified, a sample accommodating the examination object in the substantially transparent medium solution is prepared.

Thus, the examination object can be examined with high precision by detecting, outside the gelled or solidified medium solution, light emitted from the examination object in this sample. In addition, the dispersion of the medium solution due to mixing with the liquid can be prevented by bringing the liquid into contact with the surface of the medium solution in a state where the liquid is in the form of mist, bubbles, or liquid droplets with a volume smaller than that of the medium. Also, because this aspect is realized by the simple work of merely bringing the liquid into contact with the surface of the medium solution containing the examination object, sample preparation can be automated. Therefore, it is possible not only to easily prepare a sample that enables highly precise examination and imaging of the examination object with a microscope but also to easily automate the preparation of such a sample .

In the method for preparing a sample for microscope examination according to the above-described aspect, the liquid may be atomized by ultrasound radiation or pressure application.

With this configuration, the liquid can be easily atomized by a general method.

In the method for preparing a sample for microscope examination according to the above-described aspect, the medium solution may be supported by a base material in a state where the surface of the medium solution with which the liquid is brought into contact is exposed.

With this configuration, the medium solution can be brought into contact with the liquid such that the medium solution is maintained by the base material in a stable position and orientation.

In the method for preparing a sample for microscope examination according to the above-described aspect, the base material may have a shape of a rod, cup, well, ring, swab, sponge, porous body, or plate that is flat or that has irregularities on a surface thereof.

In the method for preparing a sample for microscope examination according to the above-described aspect, the base material may be made to support the medium solution containing the examination object such that the medium solution is adhered, in the form of a water droplet, to a surface of the base material.

With this configuration, the medium solution can be maintained by means of surface tension using the base material having a simple shape.

In the method for preparing a sample for microscope examination according to the above-described aspect, the base material may be made to support a droplet of the medium solution containing the examination object such that the droplet is hung.

With this configuration, the position of the examination object can be stabilized by gravity in the droplet of the medium solution while still keeping the droplet of the medium solution separated from, for example, a well.

In the method for preparing a sample for microscope examination according to the above-described aspect, the examination object may be a material that is derived from a living organism and that is formed of a cell, cell aggregate, cell tissue, spheroid, or organoid, and the examination object may be a material that is derived from an abiological object and that has fluorescence, luminescence, phosphorescence, or a dye.

In the method for preparing a sample for microscope examination according to the above-described aspect, the medium solution may be gelled or solidified after the material that is derived from a living organism and that is contained in the medium solution takes a form for examination.

With this configuration, the material that is derived from a living organism and that has taken a shape suitable for examination can be examined as the examination object.

In the method for preparing a sample for microscope examination according to the above-described aspect, the medium solution may be a culture medium that contains sodium alginate and that can grow the material derived from a living organism, and the liquid may be an aqueous solution containing divalent metal ions.

With this configuration, a medium solution that is less costly and easily available can be used as the medium solution to be gelled or solidified, and furthermore, examination is possible while growing the material derived from a living organism. In addition, it is not necessary to transfer the grown material derived from a living organism, enabling low-cost screening as a result of enhanced throughput.

In the method for preparing a sample for microscope examination according to the above-described aspect, the medium solution may be gelled or solidified up to the vicinity of a periphery of the examination object.

With this configuration, it is possible to prepare a sample in which the position of the examination object is fixed in the gelled or solidified, substantially transparent medium solution.

In the method for preparing a sample for microscope examination according to the above-described aspect, only an exposed surface, of the medium solution, with which the liquid is brought into contact may be gelled or solidified.

With this configuration, it is possible to prevent spilling or drying of the medium solution containing the examination object.

A second aspect of the present invention is a sample preparation kit including: a base material for supporting a medium solution in a state where the medium solution contains an examination object; and liquid contacting means for bringing a liquid for gelling or solidifying the medium solution into contact with a surface of the medium solution supported by the base material in a state where the liquid is in the form of mist, bubbles, or liquid droplets with a smaller volume than the volume of the medium solution.

According to this aspect, the medium solution can be easily and reliably gelled or solidified merely by bringing, using the liquid contacting means, the liquid in the form of mist, bubbles, or liquid droplets with a volume smaller than that of the medium solution into contact with the surface of the medium solution in a state where the medium solution containing the examination object is supported by the base material, thereby making it possible to prepare a sample accommodating the examination object in the substantially transparent medium solution. Therefore, it is possible not only to easily prepare a sample that enables highly precise examination and imaging of the examination object with a microscope but also to easily automate preparation of such a sample.

### {Advantageous Effects of Invention}

The method for preparing a sample for microscope examination and the sample preparation kit according to the present invention afford an advantage in that it is possible not only to easily prepare a sample that enables highly precise examination and imaging of an examination object with a microscope but also to easily automate the preparation of such a sample.

### {Brief Description of Drawings}

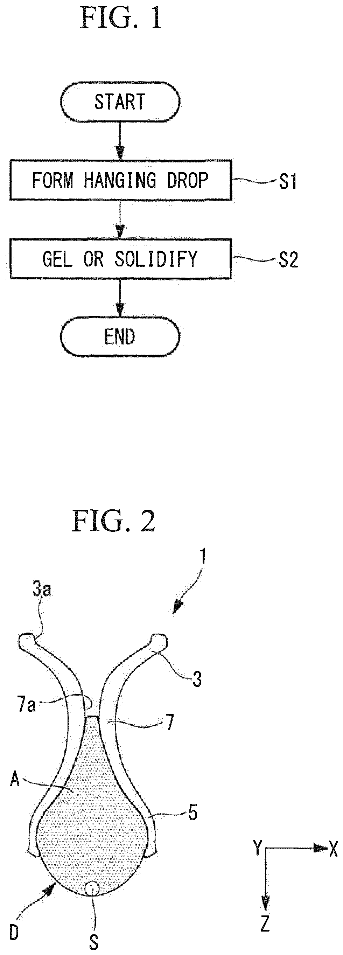

{Fig. 1}
   Fig. 1 is a flowchart for illustrating a method for preparing a sample for microscope examination according to one embodiment of the present invention.
{Fig. 2}
   Fig. 2 is a longitudinal sectional view of a hanging-drop forming tool used in the method for preparing a sample for microscope examination in Fig. 1.
{Fig. 3}
   Fig. 3 is a diagram showing a situation in which a liquid for gelling a droplet of a medium solution supported by the hanging-drop forming tool in Fig. 2 is brought into contact with this droplet, with the liquid being in the form of mist.
{Fig. 4}
   Fig. 4 is a plan view showing one example of a gelling device used in the method for preparing a sample for microscope examination in Fig. 1.
{Fig. 5A}
   Fig. 5A is a diagram showing a 90° rotated view of hanging drops that are gelled while being supported by hanging-drop forming tools.
{Fig. 5B}
   Fig. 5B is a magnified view of the vicinity of a spheroid in Fig. 5A
{Fig. 5C}
   Fig. 5C is a fluoroscopic view of the spheroid in Fig. 5B.
{Fig. 5D}
   Fig. 5D is another fluoroscopic view of the spheroid in Fig. 5B.
{Fig. 6}
   Fig. 6 is a longitudinal sectional view showing a situation in which a spheroid contained in a gelled hanging drop supported by a hanging-drop forming tool is irradiated with excitation light to generate fluorescence.
{Fig. 7A}
   Fig. 7A is a longitudinal sectional view showing a situation in which medium solutions are stored in micro wells.
{Fig. 7B}
   Fig. 7B is a longitudinal sectional view showing a situation in which a liquid for gelling the medium solutions in Fig. 7A is brought into contact with these medium solutions, with the liquid being in the form of mist.
{Fig. 7C}
   Fig. 7C is a longitudinal sectional view showing a situation in which exposed surfaces of the medium solutions in Fig. 7B are gelled.
{Fig. 8A}
   Fig. 8A is a perspective view showing a situation in which medium solutions are adhered to a slide glass in the form of water droplets.
{Fig. 8B}
   Fig. 8B is a perspective view showing a situation in which a liquid for gelling the water droplets of the medium solutions in Fig. 8A is brought into contact with these water drops, with the liquid being in the form of mist.
{Fig. 8C}
   Fig. 8C is a perspective view showing a situation in which the exposed surfaces of the water droplets of the medium solutions in Fig. 8B are gelled.
{Fig. 9A}
   Fig. 9A is a perspective view showing a situation in which a sheet-shaped biological sample containing a medium solution is disposed on a slide glass.
{Fig. 9B}
   Fig. 9B is a perspective view showing a situation in which a liquid for gelling a medium solution is brought into contact with the biological sample in Fig. 9A, with the liquid being in the form of mist.
{Fig. 9C}
   Fig. 9C is a perspective view showing a situation in which the surface of the biological sample in Fig. 9B is gelled.
{Fig. 10A}
   Fig. 10A is a perspective view showing a situation in which a cell culture sponge is moistened with a medium solution.
{Fig. 10B}
   Fig. 10B is a perspective view showing a situation in which a liquid for gelling the medium solution in Fig. 10A is brought into contact with this medium solution, with the liquid being in the form of mist.
{Fig. 10C}
   Fig. 10C is a perspective view showing a situation in which the droplet of the medium solution in Fig. 10B is gelled.

### {Description of Embodiments}

A method for preparing a sample for microscope examination and a sample preparation kit according to one embodiment of the present invention will now be described with reference to the drawings.

As shown in the flowchart of Fig. 1 and Figs. 2 and 3, the method for preparing a sample for microscope examination according to this embodiment includes: a step S1 of causing a droplet of a medium solution A, which is transparent when having been gelled or solidified, to contain at least one spheroid (examination object, material derived from a living organism) S and forming a hanging drop D in the form of a hanging droplet of the medium solution A; and a step S2 of mistily bringing a liquid B for gelling or solidifying the medium solution A into contact with the surface of the hanging drop D to gel or solidify the hanging drop D via chemical reaction.

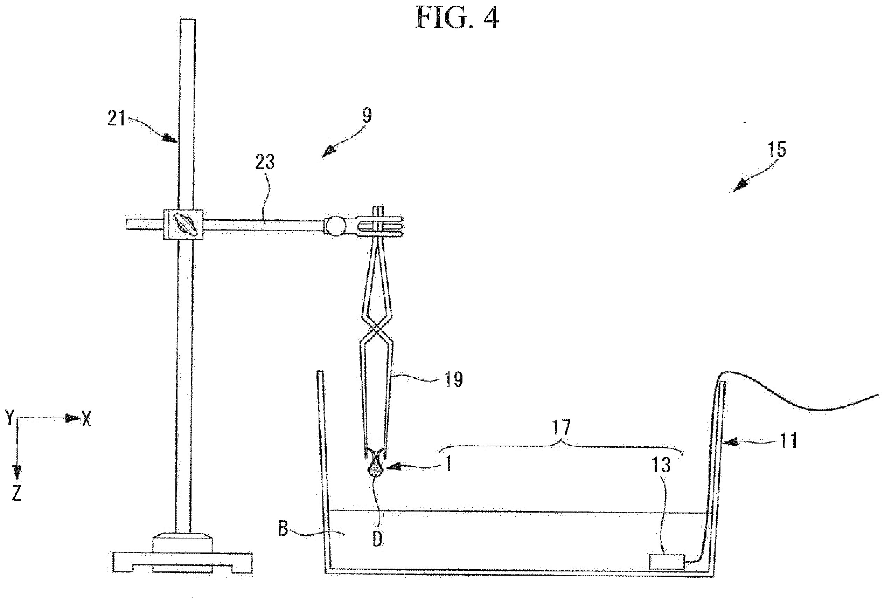

As shown in, for example, Fig. 4, this method for preparing a sample for microscope examination uses a gelling device 15 including: a hanging-drop forming tool (base material) 1 that forms the hanging drop D and that supports the formed hanging drop D; a support tool 9 for supporting the hanging-drop forming tool 1; a container 11 for storing the liquid B; and an ultrasound atomization device (liquid contacting means) 13 for bringing the liquid B into contact with the hanging drop D. A sample preparation kit 17 for preparing a sample is configured from the hanging-drop forming tool 1 and the ultrasound atomization device 13.

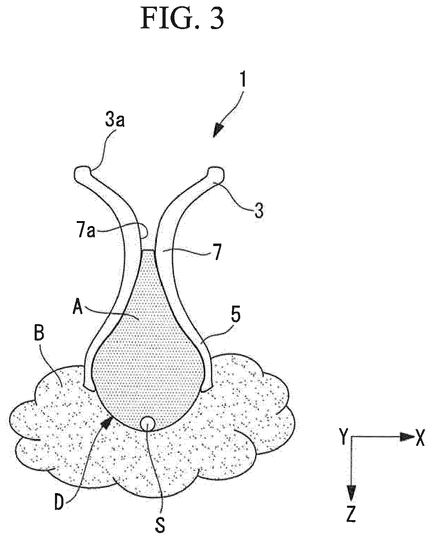

As shown in Figs. 2 and 3, the hanging-drop forming tool 1 includes: a hollow section 3 into which the medium solution A is poured; a hanging drop formation compartment 5 that supports a droplet of the medium solution A poured in the hollow section 3 such that the droplet is hung while still containing the spheroid S; and a thin conduit 7 for connecting the hollow section 3 and the hanging drop formation compartment 5.

The hollow section 3 has an opening 3a that opens on the opposite side from the conduit 7. This hollow section 3 is formed in a substantially conical shape that extends from the opening 3a so as to become narrower towards the conduit 7 in a tapered manner.

The hanging drop formation compartment 5 is formed in a substantially conical shape that extends from the conduit 7 so as to gradually widen in radially outward directions. This hanging drop formation compartment 5 supports a droplet of the medium solution A such that the bottom surface of the droplet is exposed.

The conduit 7 has a through-hole 7a that passes through from the hollow section 3 to the hanging drop formation compartment 5.

This hanging-drop forming tool 1 is disposed slightly above the liquid B in the container 11 while being supported by the support tool 9 in an orientation in which the hollow section 3 is at an upper position in the vertical direction and the hanging drop formation compartment 5 is at a lower position in the vertical direction. Hereinafter, the vertical direction is defined as a Z direction, and the directions that are orthogonal to the Z direction and that are orthogonal to each other are defined as an X direction and a Y direction.

The support tool 9 includes: a tool pincher 19 for gripping the hanging-drop forming tool 1; a stand 21 extending along the vertical direction; and a clamp 23 that extends from the stand 21 in a direction intersecting the vertical direction and that grips the tool pincher 19.

The ultrasound atomization device 13 is disposed in the liquid B in the container 11. This ultrasound atomization device 13 can atomize the liquid B into minute mist by generating ultrasound to fill the container 11 with the generated liquid B in the form of mist.

As the medium solution A, for example, a culture medium that contains sodium alginate and that can grow a material derived from a living organism is used. The medium solution A is transparent when it is gelled. The medium solution A is not limited to sodium salt, as long as it is an alginate. In addition, sodium alginate is preferably in an amount of 0.5 weight% or more, but the amount thereof is not limited to this. This medium solution A has a specific gravity of 1, which is smaller than the specific gravity of the spheroid S.

As the liquid B, for example, a calcium chloride aqueous solution, which is an aqueous solution containing divalent metal ions, such as calcium, magnesium, and strontium, is used. Calcium chloride preferably has a mol concentration of 100 mM or more, but the mol concentration is not limited to this.

The operation of the method for preparing a sample for microscope examination and the sample preparation kit 17 with the above-described configuration will be described below.

When a sample for microscope examination is to be prepared using the sample preparation method and the sample preparation kit 17 according to this embodiment, the medium solution A is first dispensed from above into the hollow section 3 of the hanging-drop forming tool 1. The medium solution A dispensed in the hollow section 3 moves down by gravity via the through-hole 7a of the conduit 7 and is then supported by the hanging drop formation compartment 5 in the form of a hanging droplet.

Thereafter, via a lower portion of the droplet of the medium solution A, which is supported by the hanging drop formation compartment 5 in a hanging manner, the spheroid S is inserted into the droplet. By doing so, the hanging drop D, which contains the spheroid S and which is a hanging droplet of the medium solution A, is formed (step S1), as shown in Fig. 2.

Because the medium solution A forming the hanging drop D has a smaller specific gravity than the spheroid S, the spheroid S moves by gravity along the boundary surface of the hanging drop D and then settles in the vicinity of the lowest point of the hanging drop D. Therefore, the spheroid S in the droplet can be made to settle at constant positions not only in the X and Y directions but also in the Z direction by predetermining the amount of the medium solution A to be dispensed in the hollow section 3. In addition, using the hanging-drop forming tool 1 eliminates the need for inverting the droplet of the medium solution A in order to form the hanging drop D.

Next, as shown in Fig. 4, the hanging drop D is disposed above the liquid B in the container 11 by supporting, with the support tool 9, the hanging-drop forming tool 1 in which the hanging drop D has been formed. In addition, the liquid B is atomized by operating the ultrasound atomization device 13. Thereafter, the container 11 is filled with the liquid B in the form of mist, and the liquid B is mistily brought into contact with the surface of the hanging drop D, as shown in Fig. 3.

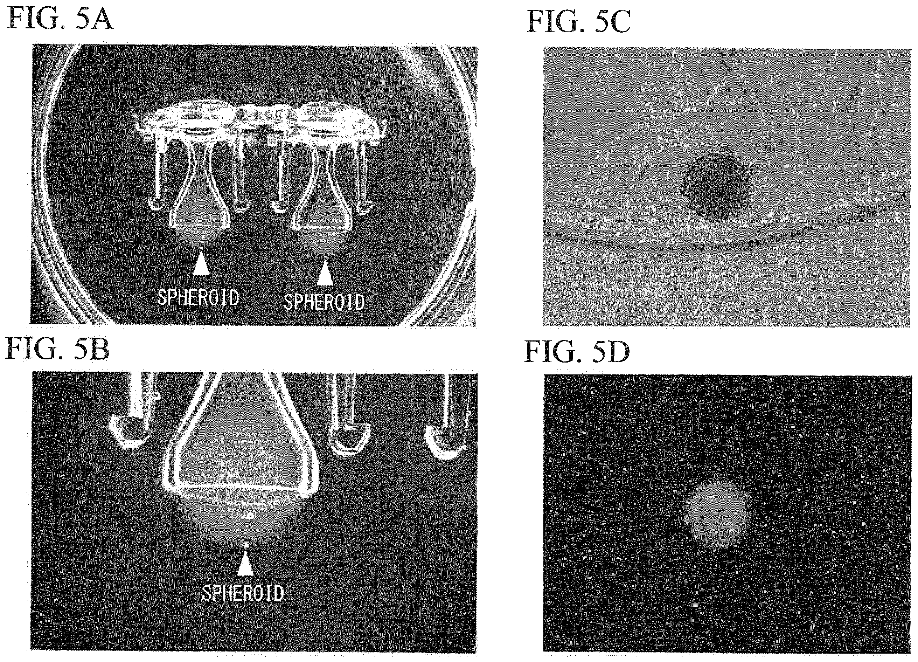

The hanging drop D is left as is at a standstill for 30 minute in the liquid B in the form of mist to let the liquid B into the hanging drop D via the exposed bottom surface thereof, thereby gelling the hanging drop D up to the vicinity of the periphery of the spheroid S (step S2). By doing so, a sample including the spheroid S that is present in the substantially transparent hanging drop D and that is fixed at the position of the lowest portion of the hanging drop D is prepared, as shown in, for example, Figs. 5A to 5D.

As described above, according to the method for preparing a sample for microscope examination and the sample preparation kit 17 of this embodiment, it is possible to easily and reliably gel the droplet of the medium solution A to prepare a sample having the spheroid S accommodated in the substantially transparent droplet merely by supporting, with the hanging-drop forming tool 1, the droplet of the medium solution A containing the spheroid S and then bringing the liquid B in the form of mist into contact with the surface of the droplet by using the ultrasound atomization device 13.

The spheroid S can also be examined with high accuracy by detecting, outside the droplet, light emitted from the spheroid S of this sample. If, for example, the droplet of the medium solution A were immersed in the solution of the liquid B, the droplet of the medium solution A would disperse as a result of being mixed with the liquid B. However, because the liquid B is mistily brought into contact with the surface of the droplet of the medium solution A, dispersion of the droplet of the medium solution A due to mixing with the liquid B can be prevented, thereby making it possible to gel the droplet of the medium solution A while preserving the shape of the hanging drop D. In addition, because this sample preparation method can be realized by the simple work of merely bringing the liquid B into contact with the surface of the droplet of the medium solution A containing the spheroid S, sample preparation can be automated. Therefore, it is possible not only to easily prepare the sample for enabling highly precise examination and imaging of the spheroid S using a microscope but also to easily automate the preparation of the sample.

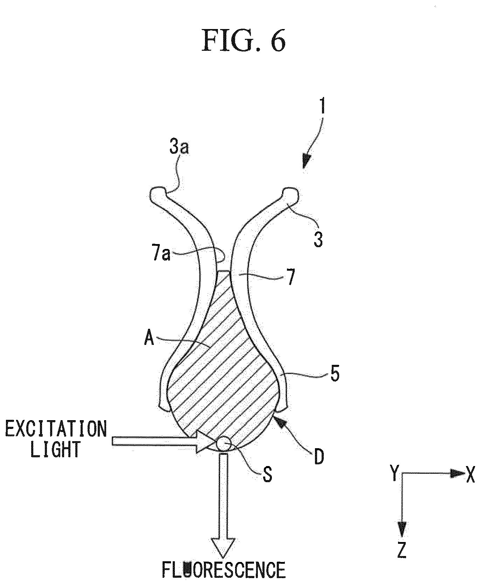

The spheroid S contained in the gelled hanging drop D can be examined using, for example, a light-sheet microscope (not shown in the figure). In this case, it is advisable that the spheroid S be irradiated with excitation light by introducing, to the hanging drop D, excitation light converged into the form of a flat surface along a plane orthogonal to the vertical direction, laterally with respect to the hanging drop D, as shown in Fig. 6. Thereafter, it is advisable that, of the fluorescence generated in the spheroid S, the fluorescence that is emitted downward in the vertical direction from the lower portion of the hanging drop D be collected by an objective lens (not shown in the figure) and detected. Here, the fluorescence generated in a wide area along the focal plane of the objective lens can be collected all at once by the objective lens by making the focal position of excitation light in the spheroid S coincide with the detection light axis and making the focal plane of the objective lens coincide with the incident plane of the excitation light, thereby making it possible to easily acquire a clear fluorescence image of the examination site in the spheroid S.

In this embodiment, the single hanging-drop forming tool 1 composed of one set of the hollow section 3, the hanging drop formation compartment 5, and the conduit 7 is used as the base material. However, a multi-well plate in which a plurality of the sets of the hollow section 3, the hanging drop formation compartment 5, and the conduit 7 are arrayed may be employed. Employing this multi-well plate easily automates dispensation, making it possible to image a large number of spheroids S for the purpose of high-throughput screening.

In addition, this embodiment may be such that, for example, the droplet of the medium solution A is made to contain cells (not shown in the figure), which are a material derived from a living organism, the operator waits until the cells take a form suitable for examination in the hanging drop D, and then the droplet of the medium solution A is gelled or solidified.

In this case, for example, in step S1, the hanging drop D may be formed by making the droplet of the medium solution A contain at least one cell, and then the spheroid S may be formed by culturing the cell in the droplet of the medium solution A. Thereafter, it is advisable that the hanging drop D be gelled or solidified by mistily bringing the liquid B into contact with the surface of the hanging drop D in step S2.

With this method, the spheroid S, which has taken a form suitable for examination, can be examined as the examination object. In addition, the work of transferring the spheroid S can be eliminated as a result of the spheroid S being formed by culturing a cell S in the hanging drop D, which leads to enhancement in the throughput and low-cost screening accordingly.

In addition, although this embodiment has been described by way of an example of the ultrasound atomization device 13 as the liquid contacting means, any means capable of atomizing the liquid B is acceptable as the liquid contacting means. The liquid contacting means may be, for example, a nebulizer for atomizing the liquid B via ultrasound and pressure application and is not limited to the ultrasound atomization device 13. In addition, instead of atomizing the liquid B, the liquid B may be in the form of, for example, bubbles or liquid droplets, such as a spray, in a volume smaller than the droplet of the medium solution A when being brought into contact with the surface of the droplet of the medium solution A. In this case, as the liquid contacting means, a spray device, a sputtering device, a pump, or the like may be employed.

In addition, although this embodiment has been described by way of an example of the spheroid S as the examination object, instead of this, a material derived from a living organism, formed of cells, a cell aggregate, cell tissue, organoid, or the like, may be employed. The cells include, for example: cells derived from vertebrates, such as human, mouse, rat, dog, monkey, rabbit, goat, cow, horse, pig, and cat; cells derived from invertebrates, such as drosophila and silkworm; fungi, such as yeast and colon bacillus; multipotent stem cells, such as ES cells and iPS cells; and stem cells, such as mesenchymal stem cells, adipose stem cells, hematopoietic stem cells, neural stem cells, hepatic stem cells, and muscle stem cells. In addition, as the examination object, a material that is derived from an abiological object and that has fluorescence, luminescence, phosphorescence, or dye may be employed.

In addition, although this embodiment has been described by way of an example of the hanging-drop forming tool 1 as the base material, any base material is acceptable, as long as the base material can support the hanging drop D so as to expose the surface of the medium solution A with which the liquid B is brought into contact. The base material may be shaped like, for example, a rod, a cup, a well, a ring, a swab, a plate, a sponge, or a porous object. A plate-shaped base material may be, for example, flat or may have irregularities on the surface thereof. In particular, the base material is preferably surface-treated so as to be capable of holding the medium solution A by adhesion.

In addition, it is advisable that the base material be formed of an inorganic substance including, for example, glass, an allied substance such as synthetic rubber, dimethylsiloxane, silicone resin, natural rubber, fluorinated polymer, polyurethane, polyethylene, polyethylene terephthalate, polyvinyl chloride, polyolefin, polycarbonate, polystyrene, polydimethylsiloxane, polysiloxane polymer, polymethyl acrylate, polymethyl hydrogen siloxane, polymethyl methacrylate, and methylhydrodienesiloxane, or an organic substance including a derivative, a friend, etc. The base material may be formed of one or more of these starting materials.

This embodiment can be modified into the following configurations.

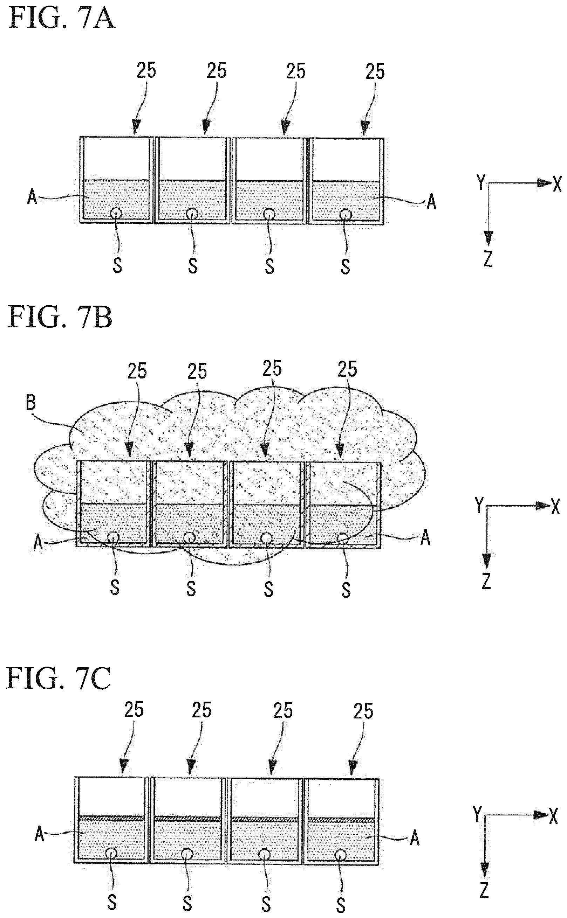

As shown in Figs. 7A to 7C, for a first modification, containers shaped like a well with a small volume, e.g., micro wells 25, may be employed as the base material. In this case, as shown in Fig. 7A, instead of the step S1 of forming the hanging drop D, it is advisable that the spheroid S and a certain amount of medium solution A be poured into each of the micro wells 25 and the surfaces of the medium solutions A be exposed from the openings of the micro wells 25.

Next, as shown in Fig. 7B, instead of the step S2 of gelling or solidifying the hanging drop D, it is advisable that the medium solutions A in the micro wells 25 be sprayed with the liquid B in the form of mist. Then, it is advisable that the exposed surfaces of the medium solutions A be gelled by mistily bringing the liquid B into contact with the exposed surfaces of the medium solutions A as shown in Fig. 7C. With this method, it is possible to prevent spilling and drying of the medium solutions A in the micro wells 25.

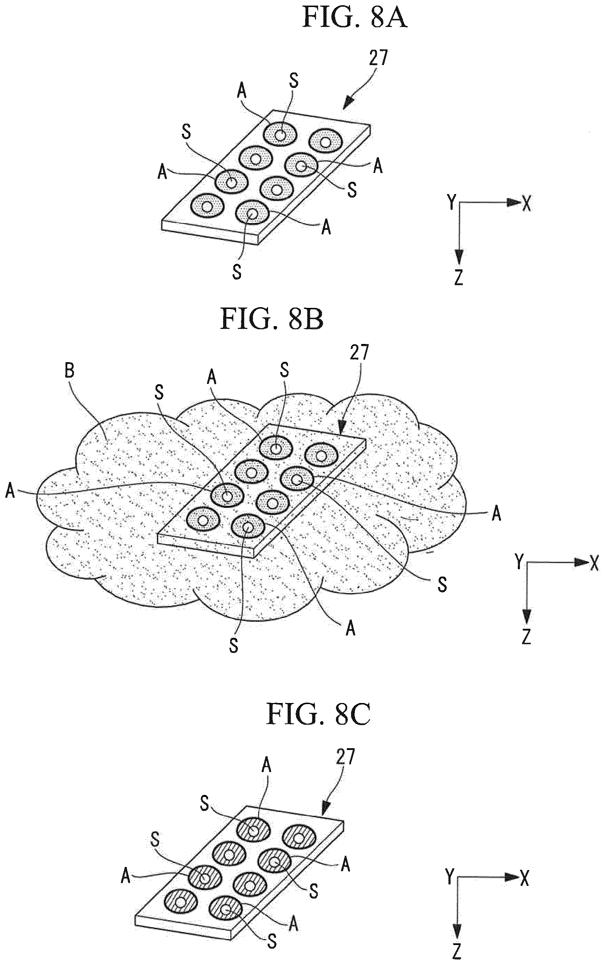

As shown in, for example, Figs. 8A to 8C, for a second modification, a slide glass 27 shaped like a flat plate or a plate having irregularities on the surface thereof may be employed as the base material. It is preferable that the surface of the slide glass 27 be hydrophobic-coated. In this case, as shown in Fig. 8A, instead of step S1, it is advisable that the medium solution A in the form of water droplets be dropped and made to adhere to one surface of the slide glass 27, the water droplets be made to contain the spheroids S, and the surfaces of the water droplets be exposed.

Next, as shown in Fig. 8B, instead of step S2, it is advisable that the droplets of the medium solution A on the slide glass 27 be sprayed with the liquid B in the form of mist. Then, as shown in Fig. 8C, it is advisable that the exposed surfaces of the droplets of the medium solution A be gelled by mistily bringing the liquid B into contact with the exposed surfaces of the droplets of the medium solution A. With this method, it is possible to prevent spilling and drying of the water droplets of the medium solution A adhered to the slide glass 27.

Although this modification has been described by way of an example where the medium solution A in the form of water droplets is gelled in a state adhered to the top surface of the slide glass 27, instead of this, the slide glass 27 to which the medium solution A is adhered in the form of water droplets may be inverted, and then the liquid B may be mistily brought into contact with the water droplets of the medium solution A that are adhered to the bottom surface of the slide glass 27 while containing the spheroids S, thereby gelling the medium solution A.

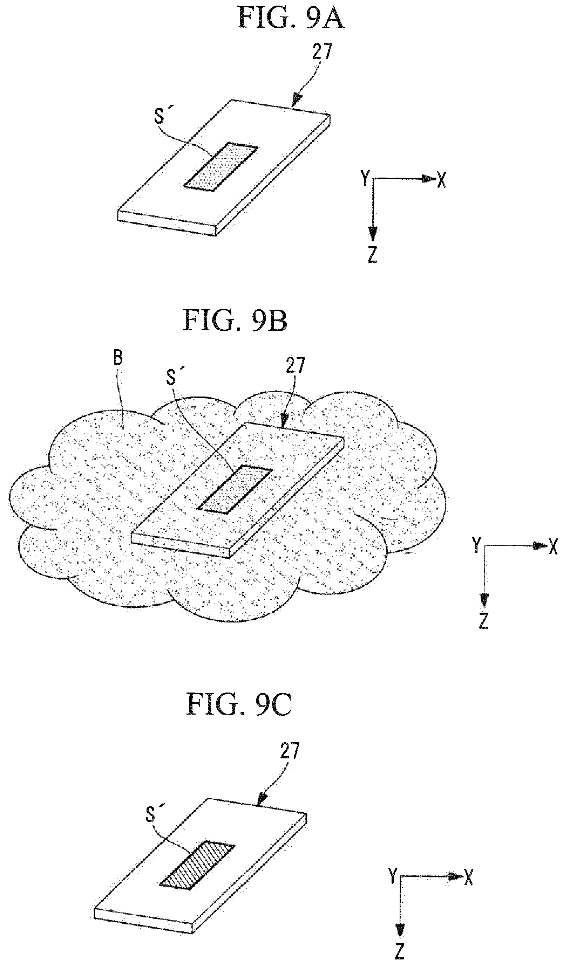

Also in this modification, a sheet-shaped biological sample (examination object) S' containing the medium solution A may be placed on the slide glass 27 as shown in, for example, Fig. 9A, and then the biological sample S' on the slide glass 27 may be sprayed with the liquid B in the form of mist as shown in Fig. 9B. As shown in Fig. 9C, with this method, it is possible to gel the surface of the sheet-shaped biological sample S' and fix the biological sample S' to the slide glass 27.

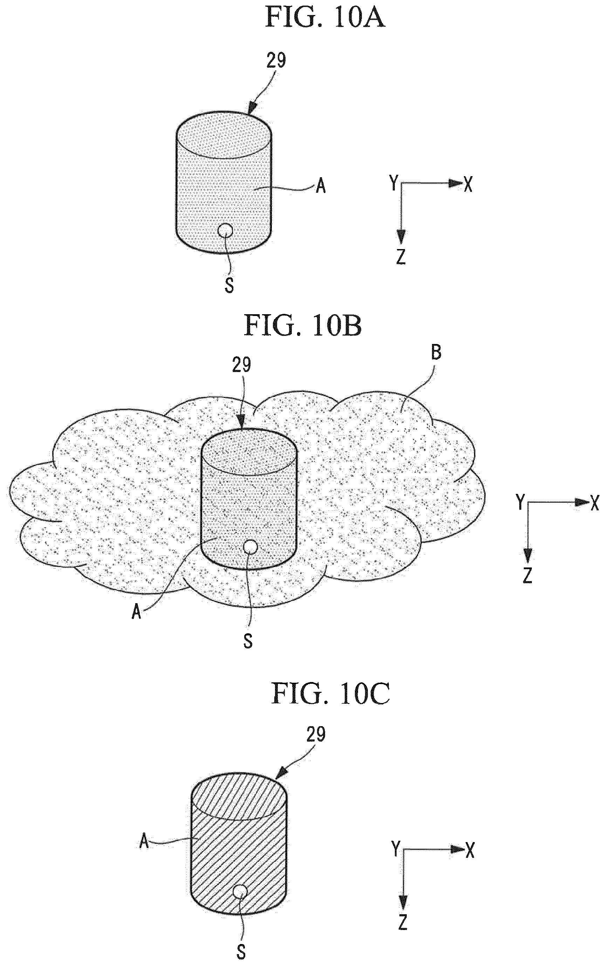

In a third modification, as the base material, an object having a spongy or porous shape may be employed, or an object formed of collagen gel may be employed, as shown in, for example, Figs. 10A to 10C. In this case, instead of the step S1, a cell culture sponge (base material) 29 may be moistened with the medium solution A containing a cell (examination object, not shown in the figure) as shown in Fig. 10A.

Next, instead of the step S2, as shown in Fig. 10B, the liquid B in the form of mist may be brought into contact with the surface of the medium solution A in the cell culture sponge 29 by spraying the cell culture sponge 29 with the liquid B in the form of mist. With this method, as shown in Fig. 10C, it is possible to fix the cell in the cell culture sponge 29 by gelling the medium solution A soaked in the cell culture sponge 29.

Although the embodiment of the present invention has been described in detail with reference to the drawings, the specific structure is not limited to those of this embodiment but includes design changes etc. that do not depart from the spirit of the present invention. The present invention is not limited to the invention applied to each of the above-described embodiments and modifications but can be applied to, for example, embodiments in which these embodiments and modifications are appropriately combined and is not particularly limited.

For example, although the above-described embodiment and the modifications thereof have been described by way of an example where an aqueous solution containing sodium alginate is employed as the medium solution A and an aqueous solution containing divalent metal ions is employed as the liquid B, other aqueous solutions may be employed for the medium solution A and the liquid B, as long as they can make the medium solution A viscoelastic, so as to be suitable for examination and measurement, by bringing the liquid B into contact with the surface of the medium solution A. In other words, any solution is acceptable for the medium solution A and the liquid B as long as the solution can gel or solidify the medium solution A by bringing the liquid B for gelling or solidifying the medium solution A into contact with the surface of the medium solution A in a state where the liquid B is in the form of mist, bubbles, or liquid droplets with a smaller volume than that of the medium solution.

### {Reference Signs List}

- 1: Hanging-drop forming tool (base material)
- 13: Ultrasound atomization device (liquid contacting means)
- 17: Sample preparation kit
- 25: Micro well (base material)
- 27: Slide glass (base material)
- 29: Cell culture sponge (base material)
- A: Medium solution
- B: Liquid
- S: Spheroid (examination object)
- S': Cell (examination object)

## Claims

1. A method for preparing a sample for microscope examination, the method comprising:
bringing a liquid for gelling or solidifying a medium solution into contact with a surface of the medium solution, which contains at least one examination object and which is substantially transparent when being gelled or solidified, in a state where the liquid is in the form of mist, bubbles, or liquid droplets with a smaller volume than the volume of the medium solution, thereby gelling or solidifying the medium solution.

2. The method for preparing a sample for microscope examination according to claim 1, wherein the liquid is atomized by ultrasound radiation or pressure application.

3. The method for preparing a sample for microscope examination according to one of claims 1 and 2, wherein the medium solution is supported by a base material in a state where the surface of the medium solution with which the liquid is brought into contact is exposed.

4. The method for preparing a sample for microscope examination according to claim 3, wherein the base material has a shape of a rod, cup, well, ring, swab, sponge, porous body, or plate that is flat or that has irregularities on a surface thereof.

5. The method for preparing a sample for microscope examination according to claim 3, wherein the base material is made to support the medium solution containing the examination object such that the medium solution is adhered, in the form of a water droplet, to a surface of the base material.

6. The method for preparing a sample for microscope examination according to claim 3, wherein the base material is made to support a droplet of the medium solution containing the examination object such that the droplet is hung.

7. The method for preparing a sample for microscope examination according to one of claims 1 to 6, wherein the examination object is a material that is derived from a living organism and that is formed of a cell, cell aggregate, cell tissue, spheroid, or organoid.

8. The method for preparing a sample for microscope examination according to one of claims 1 to 6, wherein the examination object is a material that is derived from an abiological object and that has fluorescence, luminescence, phosphorescence, or a dye.

9. The method for preparing a sample for microscope examination according to claim 7, wherein the medium solution is gelled or solidified after the material that is derived from a living organism and that is contained in the medium solution takes a form for examination.

10. The method for preparing a sample for microscope examination according to one of claims 7 and 9,
wherein the medium solution is a culture medium that contains sodium alginate and that can grow the material derived from a living organism, and
the liquid is an aqueous solution containing divalent metal ions.

11. The method for preparing a sample for microscope examination according to one of claims 1 to 9, wherein the medium solution is gelled or solidified up to the vicinity of a periphery of the examination object.

12. The method for preparing a sample for microscope examination according to one of claims 1 to 9, wherein in the medium solution, only an exposed surface with which the liquid is brought into contact is gelled or solidified.

13. A sample preparation kit comprising:
a base material for supporting a medium solution in a state where the medium solution contains an examination object; and
liquid contacting means for bringing a liquid for gelling or solidifying the medium solution into contact with a surface of the medium solution supported by the base material in a state where the liquid is in the form of mist, bubbles, or liquid droplets with a smaller volume than the volume of the medium solution.
